# EUROPEAN PATENT APPLICATION

(11) **EP 3 766 407 A1**
(43) Date of publication of application: **20.01.2021**
(21) Application number: 19766863.5
(22) Date of filing: 14.03.2019
(51) Int. Cl.: A61B 5/00, A61B 5/024, A61B 5/1455

(54) **METHOD AND DEVICE FOR DETERMINING CAUSE OF TREND IN VITAL SIGN DATA**

(30) Priority: 16.03.2018 GB 201804262; 13.12.2018 GB 201820346; 13.03.2019 KR 20190028852
(71) Applicant: Samsung Electronics Co., Ltd., Gyeonggi-do 16677 (KR)
(72) Inventor: AIL, Rohit, Gyeonggi-do. 16677 (KR); SUDHAKARAN, Sunil, Gyeonggi-do, 16677 (KR)
(74) Representative: Gulde & Partner
(86) International application number: PCT/KR2019/002954
(87) International publication number: WO 2019/177395

(57) **Abstract**

Provided is a method used by an electronic device to determine a cause of a trend in vital sign data, the method including: obtaining vital sign data of a target; determining a trend over time in the vital sign data; and determining a cause having a highest possibility of causing the determined trend, based on one or more possible causes of the determined trend and one or more stored weights for the possible cause, wherein the one or more stored weights each represent a possibility that the one or more possible causes of the determined trend is a cause responsible for the determined trend.

## Description

### TECHNICAL FIELD

Embodiments of the present disclosure relate to methods and devices for determining a cause of a trend in vital sign data, and more particularly, to methods of determining a trend in vital sign data obtained for a certain time and determining a cause of the determined trend and devices for implementing the methods.

### BACKGROUND ART

Research has been continuously conducted into systems for automatically determining whether a user has taken a certain action. For example, an automatic medicine dispenser has been developed to sense when medicine has been removed from a storage box.

Such a medicine dispenser may be used to check whether a user has taken prescribed medicine at a fixed time. However, because it may be impossible to identify whether the user has actually taken the medicine after the medicine was removed from the medicine dispenser, there may be a limit in the practical use of this device.

### DESCRIPTION OF EMBODIMENTS

### TECHNICAL PROBLEM

Research into more advanced technology for also checking whether the user has actually taken the medicine is required.

### BRIEF DESCRIPTION OF DRAWINGS

FIG. 1 is a block diagram of a device for determining a cause of a trend in vital sign data according to an embodiment of the present disclosure.
FIG. 2 is a flowchart of a method of determining a cause of a trend in vital sign data according to an embodiment of the present disclosure.
FIG. 3 is a block diagram of a system for determining a cause of a trend in vital sign data according to an embodiment of the present disclosure.
FIG. 4 is a flowchart of a method of generating each weight related to one or more possible causes of a particular trend in vital sign data according to an embodiment of the present disclosure.
FIG. 5 illustrates an example of the result of generating each weight related to one or more possible causes of a particular trend in vital sign data according to an embodiment of the present disclosure.
FIG. 6 illustrates an example of the result of determining a cause of a particular trend in vital sign data by using a weight related to one or more possible causes of a particular trend in vital sign data according to an embodiment of the present disclosure.
FIG. 7 is a flowchart of a method of determining a cause of a change in a vital sign of a target by using non-vital sign data according to an embodiment of the present disclosure.
FIG. 8 is a block diagram of a system for determining a cause of a change in vital sign data of a target according to an embodiment of the present disclosure.
FIG. 9 is a flowchart of a computer executing a method of ranking a plurality of possible causes of a particular trend in vital sign data according to an embodiment of the present disclosure.
FIG. 10 is a flowchart of a method of updating a weight related to one or more possible causes of a particular trend in vital sign data according to an embodiment of the present disclosure.

### BEST MODE

As a technical solution to the technical problem described above, according to an embodiment of the present disclosure, a method used by an electronic device to determine a cause of a trend in vital sign data includes: obtaining vital sign data of a target; determining a trend over time in the vital sign data; and determining a cause having a highest possibility of causing the determined trend, based on one or more possible causes of the determined trend and one or more stored weights for the one or more possible causes, wherein the one or more stored weights each represent a possibility that the one or more possible causes of the determined trend are the cause of the determined trend.

The determining of the cause having the highest possibility of causing the determined trend may include: transmitting information about the determined trend in the vital sign data to a server; and receiving, from the server, information about the cause having the highest possibility of causing the determined trend.

The one or more stored weights may be generated through: allocating, based on collected vital sign data and metadata related to the collected vital sign data, a weight to one or more causes of a trend in the collected vital sign data by using a machine learning algorithm; and storing the allocated weight, wherein the metadata may represent a cause of a trend in the collected vital sign data related to a plurality of individuals.

The method may further include: outputting the cause having the highest possibility of causing the determined trend on a user interface; receiving a first user input related to whether the output cause having the highest possibility is an actual cause of the trend in the vital sign data; and updating the stored weight according to the first user input.

The method may further include: receiving a second user input for confirming one of the one or more possible causes having a lower possibility than the output cause having the highest possibility, as the actual cause of the trend in the vital sign data; and updating the stored weight according to the second user input.

The one or more possible causes may include a medicine taking event when the target takes prescribed medicine.

The one or more possible causes may include a medicine taking omission event when the target has failed to take prescribed medicine, and the method may further include outputting a notification according to determining that the cause having the highest possibility of causing the determined trend in the vital sign data is the medicine taking omission event.

The method may further include, in response to determining that the cause having the highest possibility of causing the trend in the vital sign data is the medicine taking event, storing information related to vital sign data after the medicine taking event to monitor an effect of the medicine taking on the target.

The vital sign data may be obtained from a wearable electronic device including one or more vital sign sensors.

The method may further include: obtaining non-vital sign data of the target; and determining a trend over time in the non-vital sign data, wherein the determined trend in the non-vital sign data may be considered to determine the cause having the highest possibility of causing the determined trend in the vital sign data.

According to another aspect of an embodiment of the present disclosure, an electronic device for determining a cause of a trend in vital sign data includes: a memory; and one or more processors configured to: obtain vital sign data of a target; determine a trend over time in the vital sign data; and determine a cause having a highest possibility of causing the determined trend, based on one or more possible causes of the determined trend and one or more stored weights for the possible cause, wherein the one or more stored weights each represent a possibility that the one or more possible causes causing the determined trend are the cause of the determined trend.

The electronic device may further include a communication interface transmitting information about the determined trend in the vital sign data to a serve and receiving, from the server, information about the cause having the highest possibility of causing the determined trend.

The one or more stored weights may be generated through: allocating, based on collected vital sign data and metadata related to the collected vital sign data, a weight to one or more causes of a trend in the collected vital sign data by using a machine learning algorithm; and storing the allocated weight, wherein the metadata may represent a cause of a trend in the collected vital sign data related to a plurality of individuals.

The electronic device may further include a user interface outputting the cause having the highest possibility of causing the determined trend and receiving a first user input related to whether the output cause having the highest possibility is an actual cause of the trend in the vital sign data, wherein the one or more processors may update the one or more stored weights according to the first user input.

The user interface may receive a second user input for confirming one of the one or more possible causes having a lower possibility than the output cause having the highest possibility, as the actual cause of the trend in the vital sign data, and the one or more processors may update the one or more stored weights according to the second user input.

The one or more possible causes may include a medicine taking event when the target takes prescribed medicine.

The one or more possible causes may include a medicine taking omission event when the target has failed to take prescribed medicine, and the one or more processors may output a notification according to a determination that the cause having the highest possibility of causing the determined trend in the vital sign data is the medicine taking omission event.

In response to determining that the cause having the highest possibility of causing the trend in the vital sign data is the medicine taking event, the one or more processors may store information related to vital sign data after the medicine taking event to monitor an effect of taking the medicine on the target.

The vital sign data may be obtained from a wearable electronic device including one or more vital sign sensors.

The one or more processors may obtain non-vital sign data and determine a trend over time in the non-vital sign data, wherein the determined trend in the non-vital sign data may be considered to determine the cause having the highest possibility of causing the determined trend in the vital sign data.

According to another aspect of an embodiment of the present disclosure, a computer-readable recording medium stores a program that, when executed by a computer, performs the vital sign data trend cause determining method described above.

### MODE OF DISCLOSURE

The advantages and features of the present disclosure and the accomplishing methods thereof will become apparent from embodiments described below with reference to the accompanying drawings. The present disclosure may, however, be embodied in many different forms and should not be construed as being limited to the embodiments described below; rather, these embodiments of the present disclosure are provided to complete the present disclosure and fully convey the scope of the present disclosure to those of ordinary skill in the art and the present disclosure will be defined only by the scope of the claims.

Terms used herein will be briefly described and then the present disclosure will be described in detail.

The terms used herein are those general terms currently widely used in the art in consideration of functions in the present disclosure, but the terms may vary according to the intentions of those of ordinary skill in the art, precedents, or new technology in the art. Also, in some cases, there may be terms that are optionally selected by the applicant, and the meanings thereof will be described in detail in the corresponding portions of the present disclosure. Thus, the terms used herein should be understood not as simple names but based on the meanings of the terms and the overall description of the present disclosure.

Throughout the specification, when something is referred to as "including" an element, another element may be further included unless specified otherwise. Also, the term "unit" used herein may mean a software element or a hardware element such as a field-programmable gate array (FPGA) or an application-specific integrated circuit (ASIC), and the "unit" may perform some functions. However, the "- unit" is not limited to software or hardware. The "- unit" may be configured to be in an addressable storage medium or may be configured to operate one or more processors. Thus, as an example, the "- unit" may include components such as software components, object-oriented software components, class components, and task components and may include processes, functions, attributes, procedures, subroutines, segments of program code, drivers, firmware, microcode, circuits, data, databases, data structures, tables, arrays, and variables. A function provided in the elements and "units" may be combined into the smaller number of elements and "units" or may be further divided into additional elements and "units".

In the present disclosure, a device 100 for determining a cause of a trend in vital sign data may be represented the same as a user device 100 or a device 100.

Hereinafter, embodiments of the present disclosure will be described in detail with reference to the accompanying drawings so that those of ordinary skill in the art may easily implement the embodiments. Also, in order to clearly describe the present disclosure, portions irrelevant to the description will be omitted in the drawings.

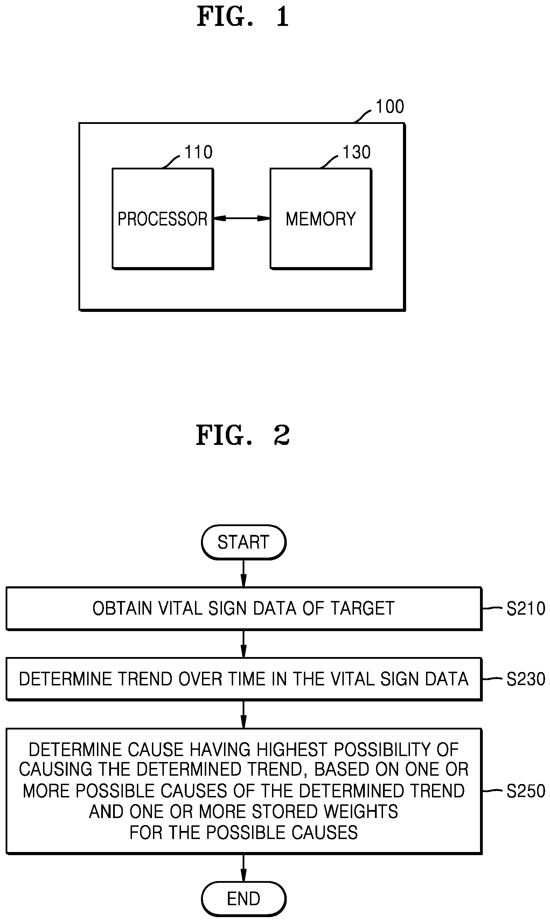

FIG. 1 is a block diagram of a device for determining a cause of a trend in vital sign data according to an embodiment of the present disclosure.

A device 100 for determining a cause of a trend in vital sign data according to an embodiment of the present disclosure may include a processor 110 and a memory 130.

The processor 110 may obtain vital sign data of a target.

The target may be a wearer or a user of the device 100 for determining the cause of the trend in the vital sign data. In an embodiment of the present disclosure, the target may be an animal.

Herein, the vital sign data may mean vital sign-related data such as pulse rate, body temperature, respiration rate, blood pressure, sleep pattern, heart rate variability (HRV), or peripheral capillary oxygen saturation (SpO₂).

The vital sign data may be obtained through various sensors inside or outside the device 100 for determining the cause of the trend in the vital sign data.

Also, the processor 110 may determine a trend over time in the vital sign data. Herein, the trend over time in the vital sign data may mean a change rate of the vital sign data or a change trend of the vital sign data for a certain time period.

For example, in an embodiment, the processor 110 may determine, as the trend over time in the vital sign data, the fact that the HRV has changed within a range of about 15 % for one hour.

Also, the processor 110 may determine the cause having the highest possibility of causing the determined trend, based on one or more possible causes causing the determined trend and one or more weights stored for the possible cause.

The possible cause causing the determined trend may include exercise such as walking or running, social activity such as telephone calling or movie viewing, food taking such as drinking or eating, or taking a prescribed medicine.

Herein, the weight for the cause that may cause the determined trend may mean each possibility that one or more possible causes stored for the determined trend will be a cause responsible for the determined trend.

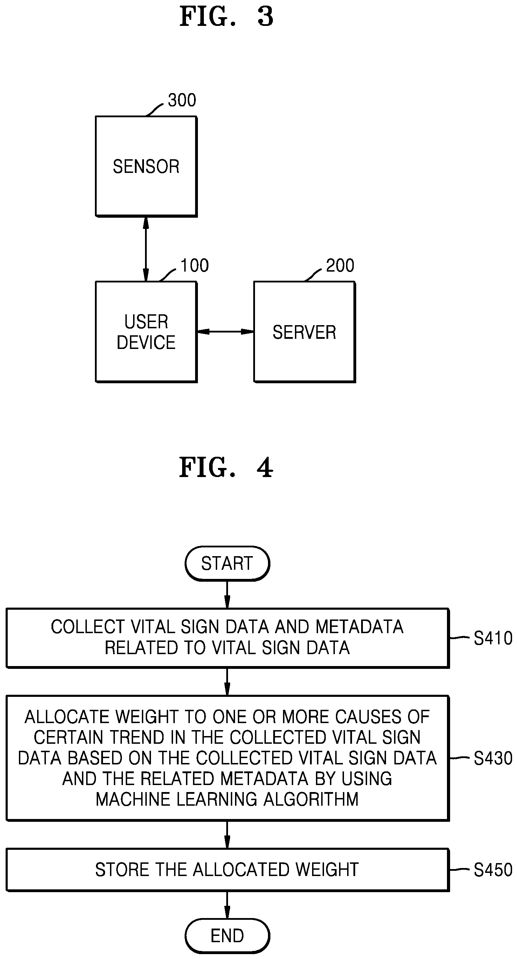

The weight for the cause that may cause the determined trend may be determined and stored based on pre-collected information, which will be described below in detail with reference to FIG. 4 or the like.

The processor 110 may determine the cause having the highest possibility of causing the determined trend, by using the weight for one or more possible causes causing the determined trend.

The memory 130 may store a program instruction or the like that causes the execution of the processor 110.

In some embodiments of the present disclosure, the device 100 for determining the cause of the trend in the vital sign data may include a plurality of memories.

In the present embodiment, the processor 110 and the memory 130 are represented as separate configuration units; however, in some embodiments of the present disclosure, the processor 110 and the memory 130 may be combined and implemented as the same configuration unit.

Also, in the present embodiment, the processor 110 and the memory 130 are represented as adjacent configuration units located inside the device 100 for determining the cause of the trend in the vital sign data; however, the processor 110 and the memory 130 may be distributed according to some embodiments because devices for performing the respective functions of the processor 110 and the memory 130 may not necessarily be physically adjacent to each other.

Also, because the device 100 for determining the cause of the trend in the vital sign data is not limited to a physical device, some of the functions of the device 100 for determining the cause of the trend in the vital sign data may be implemented by software, not by hardware.

According to some embodiments of the present disclosure, the device 100 for determining the cause of the trend in the vital sign data may further include an output device, a communication interface, and the like.

Each of the components described herein may include one or more components, and the name of the component may vary depending on the type of the device. In various embodiments, the device may be configured to include at least one of the components described herein, and some components may be omitted or other additional components may be further included. Also, some of the components of the device according to various embodiments may be combined and configured as one entity to perform the functions of the corresponding components before being combined, in the same manner.

FIG. 2 is a flowchart of a method of determining a cause of a trend in vital sign data according to an embodiment of the present disclosure.

The method illustrated in FIG. 2 may be executed in a user device 100 such as a wearable electronic device including one or more sensors for measuring a vital sign of a user wearing the device.

The method illustrated in FIG. 2 may be implemented in other types of user devices such as smart phones, tablet computers, desktop computers, or servers.

First, in operation S210, the device 100 may obtain a vital sign of a target. According to embodiments, the target may be a human or animal body in which a vital sign is measured. The vital sign data may include data related to one or more vital signs among pulse rate, body temperature, respiration rate, blood pressure, sleep pattern, and peripheral capillary oxygen saturation (SpO₂).

Also, the vital sign data may include vital sign measurement values about one or more of pulse rate, body temperature, respiration rate, blood pressure, sleep pattern, and peripheral capillary oxygen saturation (SpO₂).

In some embodiments of the present disclosure, the vital sign data may include data derived from one or more vital sign measurement values. For example, the vital sign data may include heart rate variability (HRV) data derived from the measurement value of the pulse rate.

Next, in operation S230, the device 100 may determine a trend over time in the vital sign data.

The analysis executed for determination of the trend in operation S230 may be performed in different formats according to embodiments. In some embodiments of the present disclosure, the determination of the vital sign data trend in operation S230 may be performed by applying a moving average filter to determine the average value of vital sign data over time and determining whether the determined average value of the vital sign data increases or decreases or is maintained as is.

In other embodiments, when the average value determined in operation S230 increases or decreases, the device 100 may determine that there is a change in the vital sign data; and when the determined average value is maintained as is, the device 100 may determine that the vital sign data is also stably maintained.

In some embodiments of the present disclosure, the change rate of the vital sign data may be determined in operation S230.

The analysis in operation S230 may be continuously performed as new vital sign data is obtained, or may be performed intermittently at fixed time intervals.

In operation S250, the device 100 may determine the greatest cause of the vital sign data trend according to one or more stored weights.

Each weight may relate to one or more possible causes of the determined trend and may be a factor for the possibility related to the cause responsible for the trend.

In operation S250, the most possible cause of the trend may be determined by retrieving the weight stored for one or more possible causes related to the trend determined in operation S230. In this case, among a plurality of possible causes of the determined trend, the cause having the highest weight may be determined as the most possible cause.

When the most possible cause is determined, the device 100 may take a suitable action according to the determination. For example, the device 100 may store the determination result of operation S250 for future reference by the user or a third party such as a medical expert.

In some embodiments of the present disclosure, the vital sign data may be stored in association with the most possible cause of the trend revealed in the vital sign data. In other embodiments of the present disclosure, the device 100 may automatically output a notification (alert) according to the type of the sensed cause.

FIG. 3 is a block diagram of a system for determining a cause of a trend in vital sign data according to an embodiment of the present disclosure.

In the embodiment of FIG. 3, the system for determining the cause of the trend in the vital sign data may include a device 100 for determining the cause of the trend in the vital sign data, a server 200, and one or more sensors 300.

In the embodiment of FIG. 3, the device 100 for determining the cause of the trend in the vital sign data may be a user device such as a wearable electronic device, a smart phone, a tablet computer, or a desktop computer.

The device 100 for determining the cause of the trend in the vital sign data may execute some processes in the server 200 and the sensor 300 in determining the cause of the trend in the vital sign data according to the flowchart illustrated in FIG. 2.

In operation S250 of the embodiment of FIG. 2, the weight may be stored in the user device 100 or may be stored remotely. When the weight is stored in a remote device, an operation of retrieving the stored weight may be executed remotely in an external device.

An operation of identifying the cause having the highest weight may be executed in the same device as the device for analyzing the vital sign data, or may be executed remotely in an external device.

In the embodiment of FIG. 3, the weight may be stored remotely in the server 200 that is an external device of the user device 100. The server 200 may determine the causes that may cause the determined trend, and may allocate and store the weight for each cause based on pre-collected information.

Also, the server 200 may identify the cause having the highest weight among one or more causes of the trend and transmit the same to the device 100 for determining the cause of the trend in the vital sign data.

The sensor 300 may sense various vital signs about the target and transmit the same to the user device 100. The sensor 300 may include an accelerometer, a temperature sensor, a pressure sensor, or the like.

FIG. 4 is a flowchart of a method of generating each weight related to one or more possible causes of a particular trend in vital sign data according to an embodiment of the present disclosure.

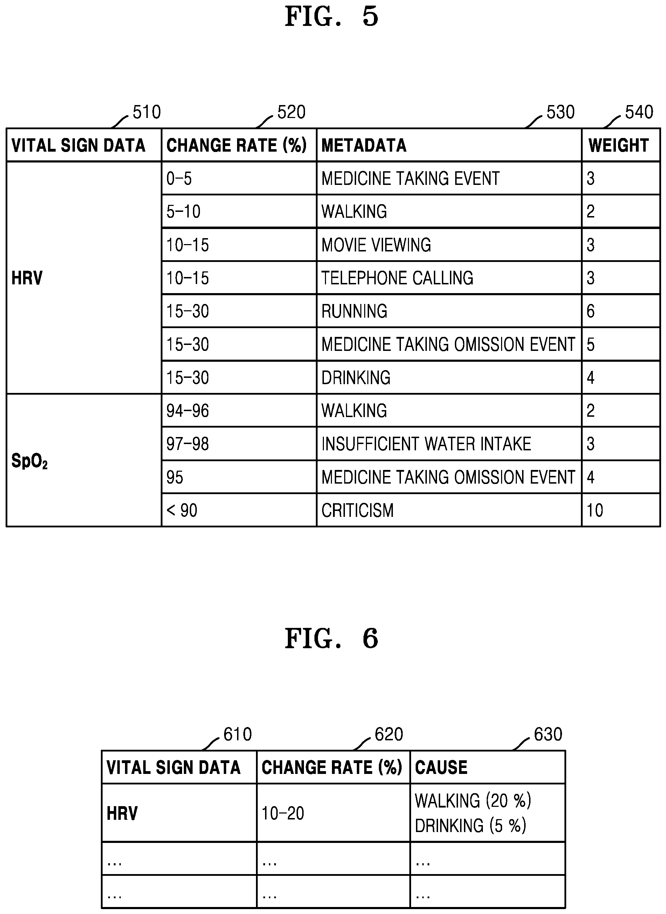

The method illustrated in FIG. 4 may be used to generate the weight, for example, by generating a table as described in FIG. 5. The operations illustrated in FIG. 4 may not need to be performed by the same device as the device executing the analysis by using the stored weight.

For example, the weight may be generated and stored remotely by the server 200 by using the method illustrated in FIG. 4. The method illustrated in FIG. 2 may be performed in a device, such as a wearable electronic device or a smart phone, capable of remote communication with a server.

In operation S410, vital sign data and metadata related to the vital sign data may be obtained for each of a plurality of individuals.

Herein, the metadata may mean a possible cause of a trend in vital sign data about a plurality of individuals. The metadata may be manually input or automatically generated.

Non-vital sign data collected simultaneously with vital sign data about a particular individual may be automatically analyzed as a type of activity performed by the individual while the data is being collected. For example, accelerometer data may be analyzed to determine that the individual is walking or running and thus may be generated as metadata such as running or walking.

In operation S430, based on the collected vital sign data and the related metadata, the device 100 or the server 200 may allocate the weight to one or more causes of a trend in the collected vital sign data by using a machine learning algorithm. For accurate weight allocation, the device 100 or the server 200 may collect vital sign data over a long period.

As described above, the allocated weights may mean each possibility for one or more causes responsible for the vital sign data trend.

Thus, the weights may be probability values. As may be seen from the example described in FIG. 5, the weights may not indicate the exact probability and may indicate the relative probability for one or more causes that may describe the current trend.

In operation S450, the weights allocated in operation S430 may be stored in a memory in association with information about one or more causes. The allocated weights may be stored in a table format as described in FIG. 5, or may be stored in other suitable formats.

Once stored, the weights may be continuously retrieved for use in the same way as illustrated in FIG. 2 and then may be used to determine the most possible cause of the vital sign data trend for a particular target.

FIG. 5 illustrates an example of the result of generating each weight related to one or more possible causes of a particular trend in vital sign data according to an embodiment of the present disclosure.

FIG. 5 illustrates an example of a structure of a database storing vital sign data 510, a trend 520 over time in the vital sign data 510, metadata 530 related to the cause that may cause the trend, and a weight 540 for each metadata 530, according to an embodiment of the present disclosure.

The metadata 530 may mean an event that may cause a particular trend. In the example described in FIG. 5, the trend is represented as a percentage of change in HRV measurement and peripheral capillary oxygen saturation (SpO₂) measurement.

The percentage of change may be determined, for example, by comparing the value read from the sensor to the last point of a particular time period and the value read from the sensor to the start point of the particular time period during the particular time period.

In some embodiments, the trend in the vital sign data may be determined by comparing the measured vital sign data and the baseline of the vital sign data about the current target. In this case, the baseline of the vital sign data may be the vital sign data value at the start point of a determined period.

As described above, the HRV may be a type of vital sign data. The peripheral capillary oxygen saturation (SpO₂) measured by using a pulse oximeter may be an example of a non-vital sign data type.

In some embodiments, the device 100 or the server 200 may be used to determine the cause of the determined trend by combining the vital sign data and the non-vital sign data.

In the example described in FIG. 5, a plurality of possible causes of the trend may include a medicine taking event and a medicine taking omission event. The medicine taking event may be an event in which the target takes a prescribed medicine. The medicine taking omission event may be an event in which the target does not take a prescribed medicine at a prescribed time.

In some embodiments, the device 100 executing the method illustrated in FIG. 2 may determine such that the time period including a scheduled medicine taking time in which the target should take a prescribed medicine may be included in the time period for determining the trend in operation S230. In this case, the most possible cause determined in operation S250 may be whether the target has taken a prescribed medication at a scheduled time.

When the most possible cause of the determined trend is determined as the medicine taking omission event, a notification may be output. The notification may be output in the form of audio, video, or tactile feedback to the user.

The notification may be used as a reminder for getting the user to take the medication. For example, the device 100 may output the notification by transmitting a notification message to a medical expert through a suitable communication method such as SMS text message or email.

In some embodiments, in response to the determination that the most possible cause of the trend in the vital sign data is the medicine taking event, the device 100 may store information related to the vital sign data after the target has taken the medicine.

The stored vital sign data may be continuously retrieved and analyzed by the device 100 and then used to monitor the effect of the medicine on the target.

In an embodiment, the device 100 may automatically sense when the target has taken the medicine and immediately record the vital sign data according to the medicine taking event, thereby allowing the user or the medical expert to check whether the medicine has a desirable effect.

When the vital sign data after the medicine taking event indicates that the medicine does not have an intended effect, the device 100 may change the dose of the medicine or prescribe another type of medicine.

FIG. 6 illustrates an example of the result of determining a cause of a particular trend in vital sign data by using a weight related to one or more possible causes of a particular trend in vital sign data according to an embodiment of the present disclosure.

FIG. 6 may indicate the result that the device 100 may output after executing all the operations of FIG. 2.

For example, the device 100 may determine that the probability of walking being the cause of the HRV being changed by about 10 % to about 20 % for a particular time is about 20 % and the probability of drinking being the same is about 5 %. In this case, the device 100 may determine walking as the cause having the highest possibility of being responsible for an about 10 % to about 20 % change of a trend 620 in vital sign data 610.

The probability value indicating the possibility of being the cause responsible for each metadata may be determined in various ways. For example, in the case of FIG. 5, a prestored weight is used to calculate the probability value.

In some embodiments, the device 100 may determine more causes as the cause that has affected the trend 620 and arrange and display the causes in order from the cause having the highest possibility of being the responsible cause to the cause having the lowest possibility of being the responsible cause.

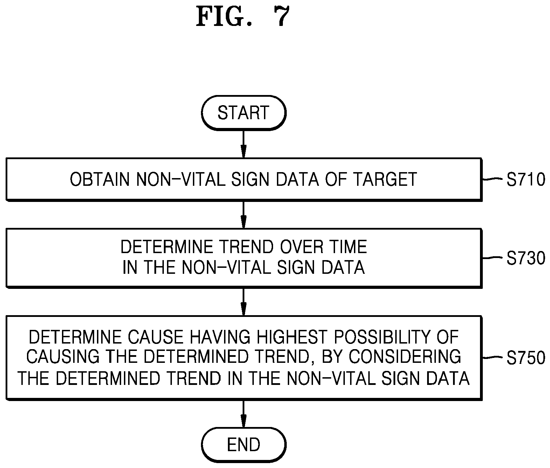

FIG. 7 is a flowchart of a method of determining a cause of a change in a vital sign of a target by using non-vital sign data according to an embodiment of the present disclosure.

As described above, in some embodiments, the non-vital sign data may be used to help determine the most possible cause of a particular trend in the vital sign data.

In operation S710, the device 100 may obtain non-vital sign data of a target.

Examples of the non-vital sign data may include SpO₂ sensor data, accelerometer data, temperature sensor data, proximity sensor data, gyroscope data, and barometric data but are not limited thereto.

In operation S730, the device 100 may determine a trend over time in the non-vital sign data. The time period used to determine the non-vital sign data trend may be the same as the time period for the vital sign data trend determined in operation S230 of the method described in FIG. 2.

In operation S750, the device 100 may use the determined non-vital sign data trend to determine the most possible cause of the determined trend in the vital sign data.

By using the non-vital sign data trend, the device 100 may improve the accuracy of the determination in operation S250 of the method described in FIG. 2.

When a plurality of possible causes are identified based on the weight stored for the vital sign data, the user device 100 may exclude some possible causes by determining whether the trend in the non-vital sign data matches the possible cause of the vital sign data trend.

Referring to the example described in FIG. 6, when the trend in the HRV change is about 15 % to about 30 %, the device 100 may identify the possible causes as "running", "medicine taking omission event", and "drinking".

For example, the device 100 may identify the trend in the non-vital sign data representing a high acceleration and deceleration pattern in operation S730 by obtaining the accelerometer data among the non-vital sign data obtainable in operation S710.

The device 100 may determine whether the trend in the non-vital sign data representing a high acceleration and deceleration pattern matches the trend in the vital sign data expected for a "running" event.

When the trend identified in operation S730 does not match the trend in the vital sign data expected for the "running" event, "running" may be excluded from the possible causes of the vital sign data trend.

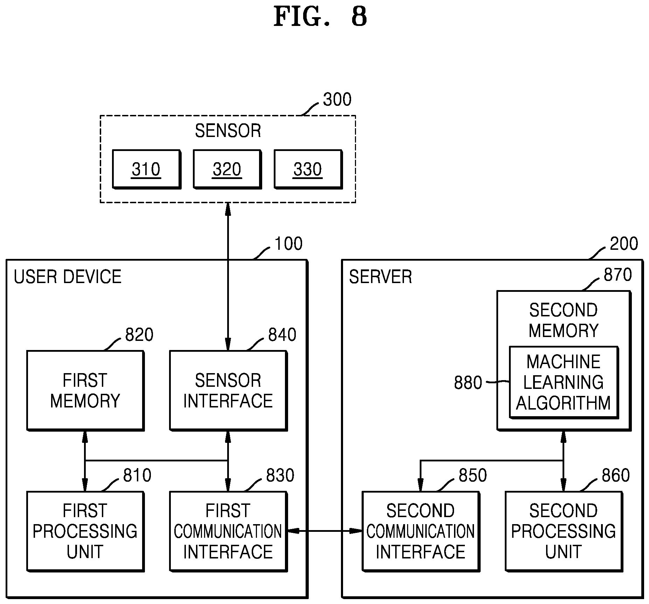

FIG. 8 is a block diagram of a system for determining a cause of a change in vital sign data of a target according to an embodiment of the present disclosure.

In the present embodiment, the system may include a user device 100 configured to determine a cause of a vital sign data change in a target and a server 200 configured to generate and store a weight related to one or more possible causes of a particular trend in vital sign data. The user device 100 and server 200 illustrated in FIG. 8 may be configured to execute the methods described above.

The user device 100 may include a first memory 820 and a first processing unit 810 including one or more processors executing computer program instructions. When executed by the first processing unit 810, the first memory 820 may be configured to store computer program instructions causing the user device 100 to execute the methods described above.

In the present embodiment, the user device 100 may further include a first communication interface 830 configured to remotely communicate with the server 200. In some embodiments, the user device 100 may store a local copy of weights related to the possible cause of the vital sign data trend, and in this case, the user device 100 may not include the first communication interface 830.

In some embodiments of the present disclosure, the user device 100 may further include a sensor interface 840 for receiving data including the vital sign data from one or more sensors 300. In the present embodiment, the sensor interface 840 may be configured to receive the vital sign data and the non-vital sign data from a plurality of sensors 310, 320, and 330.

Although three sensors such as 310, 320, and 330 are illustrated in FIG. 8, the general user device 100 may receive data from the sensors through the sensor interface 840 regardless of how many sensors are therein.

In the embodiment of FIG. 8, a plurality of sensors 300 are remote sensors that are not included in the same physical device as the user device 100.

For example, when the user device 100 is a smart phone, the sensor interface 840 may include a wired or wireless interface suitable for remotely communicating with one or more sensors 300.

In some embodiments, the user device 100 may be a wearable device coupled with one or more sensors 300, and in this case, the sensor interface 840 may not be separately included in the user device 100.

The server 200 may include a second memory 870 and a second processing unit 860 including one or more processors executing computer program instructions.

When executed by the second processing unit 860, the second memory 870 may be configured to store computer program instructions causing the server 200 to execute the methods described above.

The server 200 may further include a second communication interface 850 configured to remotely communicate with the user device 100. In some embodiments, when the user device 100 and the server 200 are implemented as the same physical device, the second communication interface 850 may not be included in the server 200.

The second memory 870 may be configured to store a machine learning algorithm 880 that may be used to allocate a weight to one or more causes of a trend in vital sign data as described in FIG. 4.

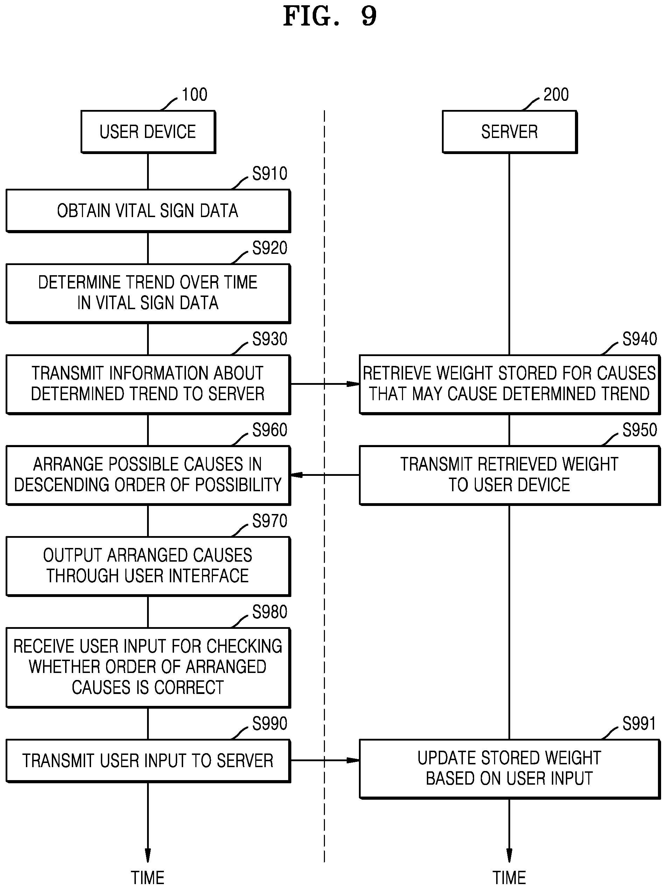

FIG. 9 is a flowchart of a computer executing a method of ranking a plurality of possible causes of a particular trend in vital sign data according to an embodiment of the present disclosure.

In the present embodiment, vital sign data may be collected and analyzed in the user device 100 capable of remote communication with the server 200 in which weights are stored.

According to the present embodiment, the user device 100 may start operation by obtaining vital sign data in operation S910 and determining a trend over time in the vital sign data in operation S920. Because operations S910 and S920 are similar to operations S210 and S230 of FIG. 2, redundant descriptions thereof will be omitted for conciseness.

In the present embodiment, in operations S930 to S960, the user device 100 may communicate with the server 200 to determine the most possible cause of the trend determined in operation S920.

In operation S930, the user device 100 may transmit information about the vital sign data trend to the server 200. In the present embodiment, the user device 100 may identify a plurality of possible causes of the trend determined in operation S920.

The server 200 may retrieve the weight stored for the causes that may cause the determined trend, in operation S940, and transmit the retrieved weight to the user device 100 in operation S950.

For example, referring to FIG. 5, when the trend determined for vital sign data of "HRV" in operation S920 is a change rate of about 15 % to about 30 %, the server 200 may retrieve possible causes of "running", "drinking", and "medicine taking omission event" and weights stored in association with the possible causes in operation S940 and transmit the retrieved weights to the user device 100 in operation S950.

In operation S960, the user device 100 may arrange the possible causes of the determined trend in descending order of possibility by using the weights stored in the server 200.

In other embodiments, the analysis executed in operation S960 may be executed by the server 200.

In an embodiment, according to the search result of the server 200, when only the weight for one possible cause is stored for the determined trend, the device 100 may determine one cause with the weight stored as the most possible cause.

In another embodiment, according to the search result of the server 200, when the weights for a plurality of possible causes are stored for the determined trend, the device 100 may determine one cause having the highest weight among the plurality of possible causes with the weights stored as the most possible cause.

In operation S970, the device 100 may output the arranged causes through a user interface. In an embodiment, only the most possible cause determined may be output to the user device 100 through the user interface. In operation S970, the user device 100 may display the possible causes, for example, on a graphical user interface (GUI).

In operation S980, the device 100 may receive a user input for check whether the order of the arranged causes is correct.

In operation S990, the device 100 may transmit the user input received in operation S980 to the server 200.

In operation S991, the server 200 may update the stored weight based on the received user input. In operation S991, the server 200 may recalculate weights related to the determined trend from the vital sign data by using a machine learning algorithm.

Because the stored weights are repeatedly updated in response to the user input, the accuracy of the stored weights may be improved with time.

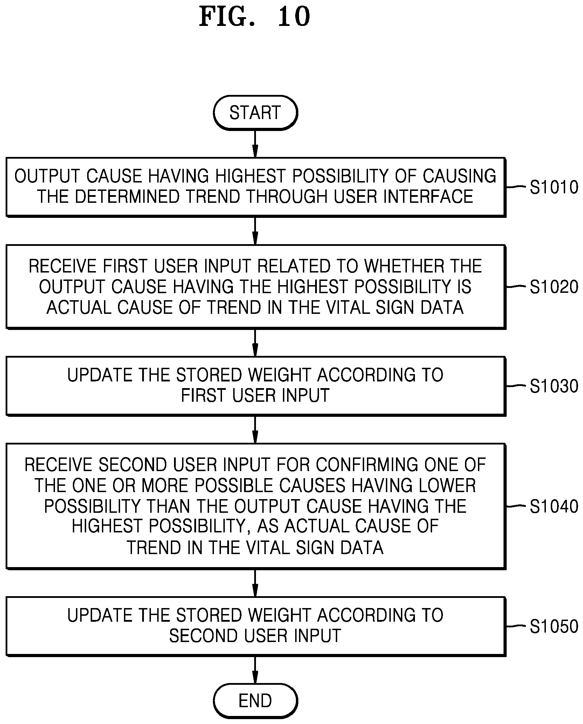

FIG. 10 is a flowchart of a method of updating a weight related to one or more possible causes of a particular trend in vital sign data according to an embodiment of the present disclosure.

In operation S1010, the device 100 may output the cause having the highest possibility of causing the determined trend through the user interface. This operation may be similar to operation S960 of FIG. 9.

In operation S1020, the device 100 may receive a first user input about whether the cause output as the most possible cause is the actual cause of the determined trend.

For example, the user device 100 may display a pop-up to allow the user to select and input "yes" or "no" as to whether the cause output as the most possible cause matches the actual cause thereof.

In operation S1030, according to the received first user input, the user device 100 may update the stored weights by transmitting the first user input to the server 200.

When the user device 100 stores the weights, the user device 100 may directly update the stored weights.

For example, the weight related to the most possible cause may be reduced in response to the first user input indicating that the most possible cause is not the actual cause of the determined trend in the vital sign data.

When the first user input indicating that the cause output as the most possible cause does not match the actual cause is received in operation S1020, the user device 100 may subsequently receive a second user input through the user interface in operation S1040.

The second user input may be an input for selecting one of a plurality of possible causes having a lower possibility than the cause determined to be most possible for the vital sign data trend.

The second user input may be a particular one of the possible causes matching the user's actual activity. The second user input may be distinguished from the first user input that is input simply as yes/no.

For example, the user device 100 may receive the second user input in the form of receiving an input for selecting one of several causes through a drop-down list from the user or in the form of receiving an input freely described in a text field.

In operation S1050, the user device 100 may update the stored weight according to the second user input. In an embodiment, the user device 100 may update the stored weights by transmitting the second user input to the server 200.

When the user device 100 stores the weights, the user device 100 may directly update the stored weights.

For example, when the second user input is received, the weight for the cause matching the cause received in the second user input may be increased.

Meanwhile, the present embodiment may be implemented by storing computer-readable code in a computer-readable storage medium. The computer-readable storage medium may include any type of storage device that may store data readable by a computer system.

The computer-readable code may be configured to perform operations for implementing the method according to the present embodiment, when read from the computer-readable storage medium and executed by a processor. The computer-readable code may be implemented in various programming languages. Also, functional programs, code, and code segments for implementing the present embodiments may be easily programmed by those of ordinary skill in the art to which the present embodiment belongs.

Examples of the computer-readable storage media may include ROM, RAM, CD-ROM, magnetic tapes, floppy disks, and optical data storage devices and may also include implementation in the form of a carrier wave (e.g., transmission through the Internet). Also, the computer-readable storage medium may be distributed over a networked computer system such that that the computer-readable code may be stored and executed in a distributed manner.

The foregoing is illustrative of embodiments of the present disclosure, and those of ordinary skill in the art will readily understand that various modifications may be made therein without materially departing from the spirit or features of the present disclosure. Therefore, it is to be understood that the embodiments described above should be considered in a descriptive sense only and not for purposes of limitation. For example, each component described as a single type may also be implemented in a distributed manner, and similarly, components described as being distributed may also be implemented in a combined form.

The scope of the present disclosure is defined not by the above detailed description but by the following claims, and all modifications or differences within the scope should be construed as being included in the scope of the present disclosure.

## Claims

1. A method used by an electronic device to determine a cause of a trend in vital sign data, the method comprising:
obtaining vital sign data of a target;
determining a trend over time in the vital sign data; and
determining a cause having a highest possibility of causing the determined trend, based on one or more possible causes of the determined trend and one or more stored weights for the possible cause,
wherein the one or more stored weights each represent a possibility that the one or more possible causes of the determined trend are the cause of the determined trend.

2. The method of claim 1, wherein the determining of the cause having the highest possibility of causing the determined trend comprises:
transmitting information about the determined trend in the vital sign data to a server; and
receiving, from the server, information about the cause having the highest possibility of causing the determined trend.

3. The method of claim 1, wherein the one or more stored weights are generated through:
allocating, based on collected vital sign data and metadata related to the collected vital sign data, a weight to one or more causes of a trend in the collected vital sign data by using a machine learning algorithm; and
storing the allocated weight,
wherein the metadata represents a cause of a trend in the collected vital sign data related to a plurality of individuals.

4. The method of claim 1, further comprising:
outputting the cause having the highest possibility of causing the determined trend on a user interface;
receiving a first user input related to whether the output cause having the highest possibility is an actual cause of the trend in the vital sign data; and
updating the one or more stored weights according to the first user input.

5. The method of claim 4, further comprising:
receiving a second user input for confirming one of the one or more possible causes having a lower possibility than the output cause having the highest possibility, as the actual cause of the trend in the vital sign data; and
updating the one or more stored weights according to the second user input.

6. The method of claim 1, wherein the one or more possible causes include a medicine taking event when the target takes prescribed medicine.

7. The method of claim 1, wherein the one or more possible causes include a medicine taking omission event when the target has failed to take prescribed medicine, and
the method further comprises outputting a notification according to a determination that the cause having the highest possibility of causing the determined trend in the vital sign data is the medicine taking omission event.

8. The method of claim 6, further comprising, in response to determining that the cause having the highest possibility of causing the trend in the vital sign data is the medicine taking event, storing information related to vital sign data after the medicine taking event to monitor an effect of taking the medicine on the target.

9. The method of claim 1, wherein the vital sign data is obtained from a wearable electronic device including one or more vital sign sensors.

10. The method of claim 1, further comprising:
obtaining non-vital sign data of the target; and
determining a trend over time in the non-vital sign data,
wherein the determined trend in the non-vital sign data is considered to determine the cause having the highest possibility of causing the determined trend in the vital sign data.

11. An electronic device for determining a cause of a trend in vital sign data, the electronic device comprising:
a memory; and
one or more processors configure to:
obtain vital sign data of a target;
determine a trend over time in the vital sign data; and
determine a cause having a highest possibility of causing the determined trend, based on one or more possible causes of the determined trend and one or more stored weights for the possible cause,
wherein the one or more stored weights each represent a possibility that the one or more possible causes of the determined trend is a cause of the determined trend.

12. The electronic device of claim 11, further comprising a communication interface transmitting information about the determined trend in the vital sign data to a serve and receiving, from the server, information about the cause having the highest possibility of causing the determined trend.

13. The electronic device of claim 11, wherein the one or more stored weights are generated through:
allocating, based on collected vital sign data and metadata related to the collected vital sign data, a weight to one or more causes of a trend in the collected vital sign data by using a machine learning algorithm; and
storing the allocated weight,
wherein the metadata represents a cause of a trend in the collected vital sign data related to a plurality of individuals.

14. The electronic device of claim 11, further comprising a user interface outputting the cause having the highest possibility of causing the determined trend and receiving a first user input related to whether the output cause having the highest possibility is an actual cause of the trend in the vital sign data,
wherein the one or more processors are further configured to update the one or more stored weights according to the first user input.

15. A non-transitory computer-readable recording medium storing a program that, when executed by a computer, performs the method of claim 1.
